# EUROPÄISCHE PATENTSCHRIFT

(11) **EP 0 847 733 B1**
(45) Veröffentlichungstag und Bekanntmachung des Hinweises auf die Patenterteilung: **08.12.2004**
(21) Anmeldenummer: 97250367.6
(22) Anmeldetag: 05.12.1997
(51) Int. Cl.: A61F 2/06

(54) **Stent**
Stent
Stent

(30) Priorität: 10.12.1996 DE 19653720
(43) Veröffentlichungstag der Anmeldung: 17.06.1998
(73) Patentinhaber: BIOTRONIK Mess- und Therapiegeräte GmbH & Co Ingenieurbüro Berlin, 12359 Berlin (DE)
(72) Erfinder: Kranz, Curt, 10825 Berlin (DE); Müller, Heinz, 91054 Erlangen (DE)
(74) Vertreter: Eisenführ, Speiser & Partner

(56) Entgegenhaltungen:
- EP-A- 0 679 372
- WO-A-95/30384
- WO-A-96/26689
- DE-C- 4 429 380

## Beschreibung

Die Erfindung betrifft einen Stent, insbesondere Koronarstent, als intraluminales Expansionselement der im Oberbegriff des Anspruchs 1 genannten Art.

Aus der EP-B1 0 364 787 ist ein aufweitbares intraluminales Element mit mindestens einem dünnwandigen, rohrförmigen Teil (nachfolgend als Stent bezeichnet) bekannt. Die Mantelfläche des Stents ist durchbrochen netzförmig ausgebildet und weist dabei Ausnehmungen auf, die durch sich geradlinig in axialer und in Umfangsrichtung erstreckende, stegartige Elemente von geringer Materialstärke begrenzt sind. Die stegartigen Elemente bestehen aus der restlichen Rohrwandung, von der das Material im Bereich der Ausnehmungen entfernt wurde. Vier dieser stegartigen Elemente bilden zusammen mit an ihren Enden vorgesehenen Verbindungsstücken einen sogenannten "expansiblen Bereich" der Mantelfläche des Stents.

Derartige Stents werden in einem operativen Eingriff, beispielsweise zur Behebung einer Stenose, unter Einwirkung von von innen nach außen gerichteten Kräften durch einen mit Druckgas beaufschlagten schlauchförmigen Dilator expandiert. Der Stent behält dabei trotz Verformung seine Rohrform bei und weitet das durch Ablagerungen verengte Gefäß auf.

Die vorstehend beschriebenen Stents werden aus biokompatiblen Materialien wie rostfreiem Stahl, Titan oder anderen Metallen gefertigt. Titan-Stents haben sich im Hinblick auf die körperliche Verträglichkeit, die medizinischen Anwendungsmöglichkeiten und die mechanische Bearbeitbarkeit als besonders günstig erwiesen.

Die zumeist eingesetzten Metalle sind jedoch bei den für Stents angewandeten Wandstärken im wesentlichen röntgentransparent, und die aus ihnen gefertigten Stents sind damit für den Arzt nicht mittels Röntgenstrahlung erkennbar. Die Erkennbarkeit der Position der Stents mit Hilfe eines geeigneten Monitors hat sich aber als sehr wesentlich für die korrekte Handhabung erwiesen.

Aus EP-A-0 709 068 ist ein in Röntgenstrahlung sichtbarer Stent bekannt, bei dem die Röntgensichtbarkeit durch Plattierung mit einem Metall hohen Atomgewichts bzw. durch verbreiterte, beispielsweise kreisringförmige, Fortsätze an den einzelnen Enden der Maschen erreicht wird.

Aus DE-U-296 07 916 ist ein Stent mit einem röntgensichtbaren Abschnitt bekannt, der insbesondere durch Miteinander-Verschweißen zweier vorgefertigter Hohlzylinder aus röntgentransparentem bzw. röntgenreflektierendem Material hergestellt wird.

Die EP 0 679 372 beschreibt einen intravaskulären Stent mit im wesentlichen zylindrischer Konfiguration und einem radiopaken Material, dass auf eine Oberfläche des Stentgrundkörpers aufgebracht wird. Das radioopake Material kann dabei über den gesamten Umfang des Stents oder in einem bestimmten Abschnitt aufgebracht sein, so dass sich ein umlaufendes radioopakes Band bildet.

Bei beiden genannten Anordnungen ist die Genauigkeit der Lagebestimmung des Stents noch verbesserungsfähig.

Der Erfindung liegt daher die Aufgabe zugrunde, einen Stent der eingangs genannten Gattung anzugeben, dessen Position im Körper eines Patienten durch Röntgenstrahlung besonders präzise feststellbar ist.

Die Aufgabe wird durch einen Stent mit den Merkmalen des Anspruchs 1 gelöst. Die Erfindung schließt die technische Lehre ein, daß zur Röntgenerkennung eines Stents auch dann ein ausreichender Kontrast erzielbar und zugleich eine äußerst präzise Bestimmung auch der Winkellage des Stents im Körper möglich ist, wenn ein nur eng begrenzter, für die Bestimmung der Winkellage wesentlicher Bereich bzw. ein einzelnes Element röntgensichtbar ausgebildet sind. Dies ist insbesondere ein der Stent-Umfangskontur angepaßter linearer Bereich, noch spezieller ein Kreisring oder Kreisringsegment. Dessen Projektion im Röntgenbild gibt präzise Aufschluß nicht nur über den Ort, sondern auch über die Orientierung des Stents.

Zum Vorsehen dieses Bereiches bzw. Elementes sind insbesondere die Endbereiche des Stents gut geeignet. Bei bekannter Stentlänge kann bereits mit einem einzelnen röntgenopaken Element die exakte Stentposition bestimmt werden.

Von Bedeutung ist, daß durch die röntgenopake Modifizierung die Expansionsfähigkeit des Stents im wesentlichen nicht beeinträchtigt wird.

Hierzu ist das röntgenopake bzw. -reflektierende Element bzw. der entsprechende Bereich mit einer "Expansionsreserve" ausgeführt. Nach einer Ausführungsform bertehen die bogenförmig verrundeten Verbindungsstücke der stegartigen Elemente am Ende des Stents selbst aus einem röntgenopaken Werkstoff.

Für die in den vorstehend beschriebenen Ausführungsformen des erfindungsgemäßen, vorzugsweise aus Titan hergestellten Stents ist als Werkstoff für das röntgenopake Element insbesondere Gold, Silber oder Tantal vorgesehen, da diese Metalle neben relativ hohem Reflexionsvermögen im Röntgenstrahlungsbereich die erforderliche Biokompatibilität aufweisen.

Zur Herstellung des Stents und des zur Herstellung des Stents erforderlichen, als hohlzylindrisches Rohr ausgebildete Halbzeugs ist für die erforderlichen Schweißverbindungen und Austrennvorgänge - insbesondere bedingt durch die geringen Teileabmessungen - der Einsatz lasertechnischer Mittel vorgesehen.

Um die linearen bzw. (im nicht-expandierten Zustand) verrundeten Abschnitte an den Enden oder in der Mitte des Stents röntgenopak auszubilden, wird insbesondere an den Enden oder in der Mitte eines dünnwandigen Stent-Hohlzylinders (etwa aus Ti) ein aus röntgenopakem Material bestehender mäanderförmig gekrümmter Faden (etwa aus Ta) auf den Hohlzylinder auf- oder in diesen eingeschweißt. Die Materialpaarung Ti-Ta ist besonders günstig, weil diese Metalle gut ineinander löslich und daher gur miteinander verschweißbar sind.

Der erfindungsgemäße Stent weist in dieser Ausführung eine gute Körperverträglichkeit und eine ausgezeichnete Verformbarkeit auf. Eine äußere Mikrobeschichtung aus amorphen Siliciumcarbid wirkt zusätzlich einer Thrombenbildung entgegen.

Da sich der Stent beim Expandieren aufgrund der Verformung der sich axial erstreckenden stegartiges Elemente am Stentende nur in beschränktem Maße an die Form des sich in diesem Bereich, wo der Stent in seiner Wirksamkeit endet, angrenzenden Gewebes eines aufzuweitenden Blutgefäßes anpassen kann, ist entsprechend einer vorteilhaften Weiterbildung der Erfindung der Materialquerschnitt der stegartigen Elemente des Stents am Stentende verringert. Dies sichert in günstiger Weise, daß von von einem Stent erweiterten Gefäßbereich ein möglichst "fließender" Übergang mit einer variablen ausgleichenden Querschnittsänderung zu dem sich anschließenden, stenosefreien Gefäßbereich erfolgt.

Diese Querschnittsverringerung wird bevorzugt unter Beibehaltung der radialen Abmessungen in tangentialer Richtung erzeugt, so daß der Stent aus einem rohrförmigen Halbzeug mittels eines Laser-Schneidwerkzeugs durch Herausscheiden der ausgesparten Bereiche gefertigt werden kann. Bei in dieser Weise verringerter Steifigkeit der Stents zum Endbereich hin ist es von besonderer Bedeutung, daß durch angepaßte Ausbildung der röntgenopake Bereiche oder Elemente diese verringerte Steifigkeit nicht wieder heraufgesetzt wird.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: ein Ausführungsbeispiel der Erfindung in Seitenansicht,
- Figur 2: ein weiteres Ausführungsbeispiel der Erfindung in Seitenansicht sowie
- Figur 3: eine günstige Weiterbildung des in Figur 2 gezeigten Ausführungsbeispiels der Erfindung in Seitenansicht.

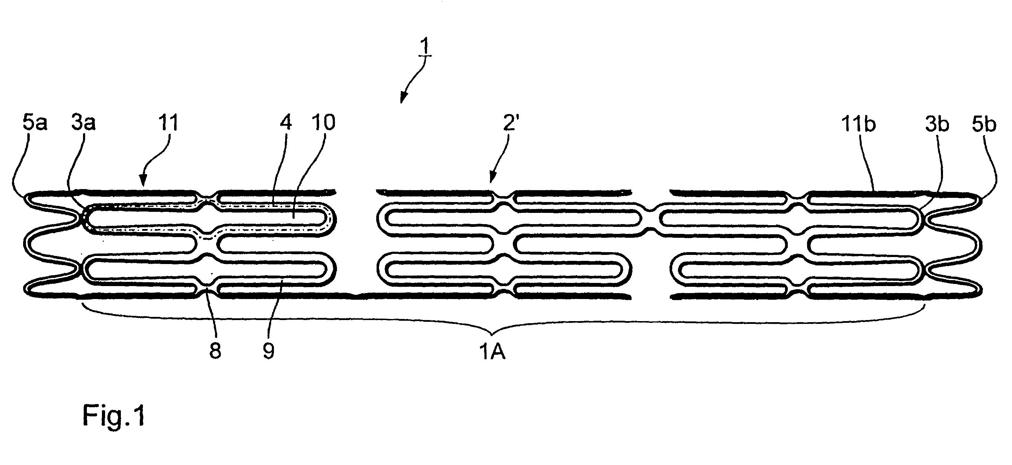

Ein in Fig. 1 skizzierter Stent 1 weist einen hohlzylindrischen Grundkörper 1A aus Titan mit zahlreichen Ausnehmungen 10 auf, welche jeweils von expansiblen Maschen umschlossen sind, die die Form von gestauchten Ringen aufweisen. Diese werden nachfolgend als "expansible Bereiche" bezeichnet und sind an einem Beispiel mit einer strichpunktierten Linie 4 markiert. Diese expansiblen Bereiche 4 werden gebildet durch umlaufende schmale stegartige Elemente 9 mit rechtekkigem Querschnitt und zeichnen sich dadurch aus, daß sie eine Ausnehmung 10 im wesentlichen ringförmig umschließen.

Die expansiblen Bereiche 4 haben in vollständig expandiertem Zustand (in der Zeichnung nicht dargestellt) nahezu die Form eines Vielecks. Sie werden bei der Herstellung (primär) derart geformt, daß sie sich nach dem Einbringen des Stents 1 in ein Gefäß durch Dilatieren mit einem Ballonkatheter mit minimaler Verformung in die Polygonalform umwandeln.

Der in Fig. 1 dargestellte Stent 1 ist in mehrere, in axialer Richtung gereihte, strukturell gleichartig ausgebildete Segmente 2 untergliedert. Sowohl zur Verbindung der expansiblen Bereiche 4 der einzelnen Stentsegmente 2 als auch der benachbarten Segmente 2 sind gleichartig ausgebildete Koppelelemente 8 vorgesehen. An beiden Enden des Hohlzylinders 1A ist der Materialquerschnitt in den Maschen und damit die Steifigkeit kontinuierlich verringert.

Der Hohlzylinder 1A weist an seinen beiden Enden 11a und 11b jeweils einen mäanderförmig konfigurierten, angeschweißten Faden 5a bzw. 5b aus einet Tantallegierung auf. Die röntgenopaken Fäden 5a, 5b sind jeweils über einzene Laserschweißpunkte mit den stirnseitigen Maschenbögen des Ti-Hohlzylinders verbunden. Bei Dilatation des Stents dehnen sich die Fäden 5a, 5b in einen gestreckten kreisringförmigen Zustand. Materialquerschnitt und Legierungszusammensetzung der Ta-Fäden sind in Anpassung an die Enden des Hohlzylinders derart gewählt, daß sie der Steifigkeitskennlinie in den Stent-Endbereicheri folgen. Die Schwächung der Stent-Endbereiche wird daher auch durch die röntgenopaken Elemente nicht beeinträchtigt.

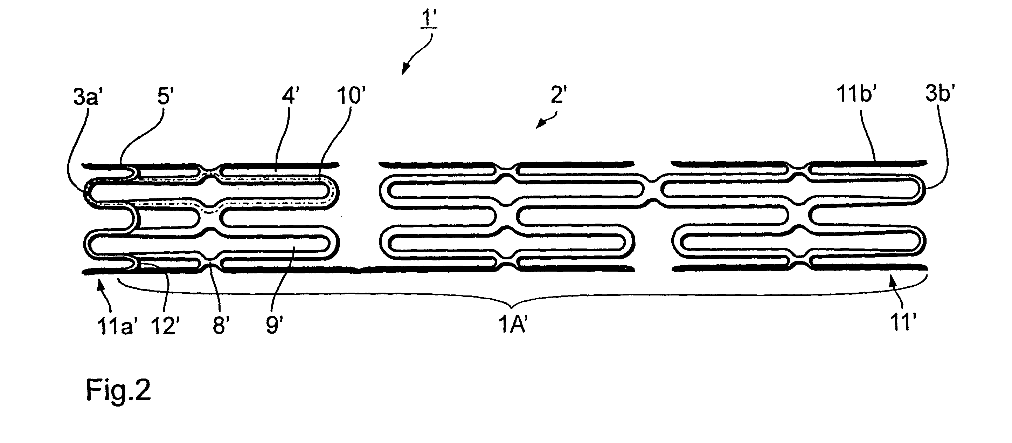

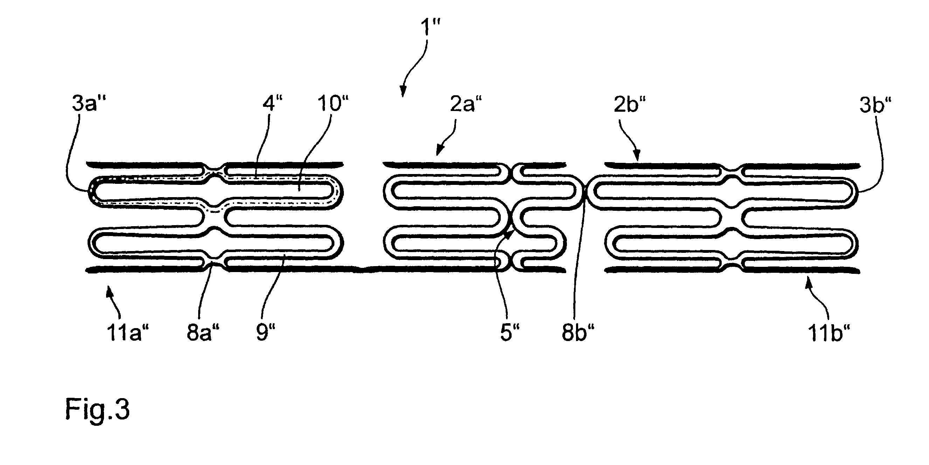

Die in Fig. 2 und 3 in Seitenansicht dargestellten Stents 1' und 1" weisen Elemente bzw. Bereiche auf, die partiell aus solchen bestehen. Da damit die Außenkontur kaum verändert wird, werden auch die Strömungseigenschaften des Stents nicht wesentlich beeinflußt. Ähnliche Elemente wie in Fig. 1 tragen dieselben Bezugsziffern wie dort.

Entsprechend Fig. 2 bestehen die bogenförmigen Abschnitte 3' der expansiblen Bereiche 4' an dem einen Ende 11a' des Stents 1' partiell aus röntgenopakem Material und sind durch zusätzliche gebogenen Stege 12', die völlig aus röntgenopakem Material bestehen, zu einem geschlossenen Markierungsfaden miteinander verbunden. Diese Konfiguration kann durch mänderförmiges Aufschweißen eines Fadens 5' aus röntgenopakem Material auf die - entsprechend geschwächt vorgefertigten - Bogenabschnitte gebildet werden. Das andere Ende 11b' des Stents 1' ist hier nicht mit einer Röntgenkennung versehen.

Bei dem Ausführungsbeispiel gemäß Fig. 3 ist ersichtlich, daß ein röntgenopaker Faden 5" im mittleren Teil des Stens 1'' eingefügt ist. Er ist im Bereich der tangential verlaufenden Stege mit den benachbarten röntgentransparenten Segmenten 2a" und 2b" verschweißt, so daß der Stent 1" insgesamt neben materialeinheitlichen Stegen 8a" auch zusammengesetzte Stege 8b" umfaßt.

Die Breite der oben angesprochenen röntgenopaken bzw. -reflektierenden Elemente oder Bereiche sollte im Interesse einer einwandfreien Erkennung im Röntgenbild mehr als 15 µm, bevorzugt mehr als 75 µm, betragen, wobei die Bedingung einzuhalten ist, daß es/er sich im wesentlichen linear erstreckt. Das Element ist im Interesse guter Durchströmungseigenschaften des Stents sowie aus fertigungstechnischen Gründen bevorzugt auf der Außenfläche des Grundkörpers anzubringen.

Zur Vermeidung von Stenosen bzw. zur Verringerung der Restenosegefahr weist der Stent eine äußere Beschichtung aus amorphem Siliciumcarbid auf, welche auch die röntgenopaken Elemente oder Bereiche überdeckt, so daß insoweit eine einheitlich äußere Oberflächenbeschaffenheit besteht.

Die Erfindung beschränkt sich in ihrer Ausführung nicht auf die vorstehend angegebenen bevorzugten Ausführungsbeispiele. Vielmehr ist eine Anzahl von Varianten günstig, welche von der dargestellten Lösung auch bei anders gearteter Ausführung Gebrauch macht.

## Patentansprüche

1. Stent (1, 1*'*, 1''), insbesondere Koronarstent, mit einem dünnwandigen, hohlzylindrischen Grundkörper (1a, 1a', 1a") aus röntgentransparentem Material, dessen Mantelfläche durch Ausnehmungen (10) durchbrochen netzförmig ausgebildet ist, und mindestens einem röntgenopaken Element oder Bereich (5a, 5b, 5', 5"), **dadurch gekennzeichnet, dass** das röntgenopake Element oder der röntgenopake Bereich (5a, 5b, 5', 5") sich in Gestalt eines mäanderförmigen Fadens um den gesamten Umfang des Stents (1; 1'; 1") erstreckt und der Faden zumindest partiell völlig aus dem röntgenopaken Material besteht.

2. Stent nach Anspruch 1 oder 2, **dadurch gekennzeichnet, daß** das röntgenopake Element bzw. der röntgenopake Bereich (5a, 5b; 5') an mindestens einem der Enden (11a, 11b; 11a') des Stents (1; 1'), insbesondere auf der Außenfläche des Grundkörpers (1A; 1A'; 1A"), vorgesehen ist.

3. Stent nach einem der vorangehenden Ansprüche, **dadurch gekennzeichnet, daß** das röntgenopake Element (5a, 5b; 5'; 5") durch Laser-Punktschweißung am röntgentransparenten Grundkörper (1A; 1A'; 1A") befestigt ist.

4. Stent nach einem der vorangehenden Ansprüche, **dadurch gekennzeichnet, daß** das röntgenopake Element oder der röntgenopake Bereich (5a, 5b; 5', 5") aus rostfreiem Stahl, Gold, Silber oder Tantal oder einer mindestens eines dieser Metalle enthaltenden Legierung besteht.

5. Stent nach Anspruch 4, **dadurch gekennzeichnet, daß** der röntgentransparente Grundkörper (1A; 1A'; 1A") im wesentlichen aus Titan und das röntgenopake Element oder der röntgenopake Bereich (5a, 5b; 5'; 5") im wesentlichen aus Tantal besteht.

6. Stent nach einem der vorangehenden Ansprüche, **dadurch gekennzeichnet, daß** der Wandungsquerschnitt des Stents (1, 1', 1") in einem an ein Ende angrenzenden Bereich (11a, 11b; 11a', 11b'; 11a", 11b") eine derartige Schwächung aufweist, daß seine Steifigkeit an diesem Ende auch unter Einschluß eines dort angebrachten röntgenopaken Elements oder Bereichs (5a, 5b; 5'; 5") verringert ist.

7. Stent nach einem der vorangehenden Ansprüche, **dadurch gekennzeichnet, daß** eine das röntgenopake Element oder den röntgenopaken Bereich einschließende Beschichtung aus amorphem Siliciumcarbid vorgesehen ist.

8. Stent nach einem der vorangehenden Ansprüche, **dadurch gekennzeichnet, daß** die Breite des röntgenopaken Elements oder Bereichs (5a, 5b; 5'; 5") mindestens 15 µm, bevorzugt mehr als 75 µm, beträgt.

## Claims

1. Stent (1, 1', 1"), in particular a coronary stent having a thin-walled, hollow-cylindrical basic body (1a, 1a', 1a") consisting of X-ray-transparent material, whose peripheral surface is formed in the manner of a net perforated by cut-outs (10), and of at least one X-ray-opaque element or region (5a, 5b, 5', 5"), **characterised in that** the X-ray-opaque element or X-ray-opaque region (5a, 5b, 5', 5") extends in the form of a meandering thread around the entire circumference of the stent (1, 1', 1") and at least part of the thread consists completely of the X-ray-opaque material.

2. Stent as claimed in claim 1 or 2 [sic], **characterised in that** the X-ray-opaque element or X-ray-opaque region (5a, 5b; 5') is provided on at least one of the ends (11a, 11b; 11a') of the stent (1; 1'), in particular on the outer surface of the basic body (1A; 1A'; 1A").

3. Stent as claimed in any one of the preceding claims, **characterised in that** the X-ray-opaque element (5a, 5b; 5'; 5") is attached by laser spot-welding to the X-ray-transparent basic body (1A; 1A'; 1A").

4. Stent as claimed in any one of the preceding claims, **characterised in that** the X-ray-opaque element or X-ray-opaque region (5a, 5b; 5', 5") consists of stainless steel, gold, silver or tantalum or of an alloy containing at least one of these metals.

5. Stent as claimed in claim 4, **characterised in that** the X-ray transparent basic body (1A; 1A'; 1A") consists substantially of titanium and the X-ray-opaque element or X-ray-opaque region (5a, 5b; 5'; 5") consists substantially of tantalum.

6. Stent as claimed in any one of the preceding claims, **characterised in that** the wall cross-section of the stent (1, 1', 1") comprises, in a region (11a, 11b; 11a', 11b'; 11a", 11b") adjoining an end, a weakened section such that its rigidity at this end is reduced even with the inclusion of an X-ray-opaque element or region (5a, 5b; 5'; 5") provided at this site.

7. Stent as claimed in any one of the preceding claims, **characterised in that** a coating consisting of amorphous silicon carbide is provided which includes the X-ray-opaque element or X-ray-opaque region.

8. Stent as claimed in any one of the preceding claims, **characterised in that** the width of the X-ray-opaque element or region (5a, 5b; 5'; 5") is at least 15 µm, preferably more than 75 µm.

## Revendications

1. Extenseur (1, 1', 1"), en particulier extenseur coronarion comportant un corps de base cylindrique creux à parois minces (1a, 1a', 1a") en matériau transparent aux rayons X, dont la surface latérale est en forme de réseau interrompu par des évidements (10), et au moins un élément ou domaine opaque aux rayons X (5a, 5b, 5', 5"), **caractérisé en ce que** l'élément opaque aux rayons X ou le domaine opaque aux rayons X (5a, 5b, 5', 5") s'étend sur toute la périphérie de l'extenseur (1, 1', 1") sous forme d'un fil en forme de méandres et le fil consiste au moins partiellement totalement en le matériau opaque aux rayons X.

2. Extenseur selon la revendication 1 ou 2, **caractérisé en ce que** l'élément opaque aux rayons X ou le domaine opaque aux rayons X (5a, 5b ; 5') est prévu à au moins l'une des extrémités (11a, 11b ; 11a') de l'extenseur (1 ; 1'), en particulier sur la surface externe du corps de base (1A ; 1A' ; 1A").

3. Extenseur selon l'une des revendications précédentes, **caractérisé en ce que** l'élément opaque aux rayons X (5a, 5b ; 5' ; 5") est fixé par soudage par points laser sur le corps de base transparent aux rayons X (1A ; 1A' ; 1A").

4. Extenseur selon l'une des revendications précédentes, **caractérisé en ce que** l'élément opaque aux rayons X ou le domaine opaque aux rayons X (5a, 5b ; 5', 5") consiste en acier inoxydable, en or, en argent ou en tantale, ou en un alliage contenant au moins l'un de ces métaux.

5. Extenseur selon la revendication 4, **caractérisé en ce que** le corps de base transparent aux rayons X (1A ; 1A' ; 1A") consiste essentiellement en titane et l'élément opaque aux rayons X ou le domaine opaque aux rayons X (5a, 5b ; 5' ; 5") consiste essentiellement en tantale.

6. Extenseur selon l'une des revendications précédentes, **caractérisé en ce que** la section droite de la paroi de l'extenseur (1, 1', 1") présente dans un domaine contigu d'une extrémité (11a, 11b ; 11a', 11b' ; 11a", 11b") un affaiblissement tel que sa rigidité est réduite au niveau de cette extrémité même avec inclusion d'un élément ou domaine opaque aux rayons X (5a, 5b ; 5' ; 5") qui y est appliqué.

7. Extenseur selon l'une des revendications précédentes, **caractérisé en ce qu'**il est prévu un revêtement en carbure de silicium amorphe qui inclut l'élément opaque aux rayons X ou le domaine opaque aux rayons X.

8. Extenseur selon l'une des revendications précédentes, **caractérisé en ce que** la largeur de l'élément ou domaine opaque aux rayons X (5a, 5b ; 5' ; 5") est d'au moins 15 µm, de préférence supérieure à 75 µm.
